# EUROPEAN PATENT APPLICATION

(11) **EP 1 792 604 A1**
(43) Date of publication of application: **06.06.2007**
(21) Application number: 06024270.8
(22) Date of filing: 23.11.2006
(51) Int. Cl.: A61K 8/37, A61K 8/41, A61Q 5/00, A61Q 5/12

(54) **Hair treatment composition**

(30) Priority: 02.12.2005 JP 2005349182
(71) Applicant: Cognis IP Management GmbH, 40589 Düsseldorf (DE)
(72) Inventor: Masaki, Koichi, Ibaraki 306-0213 (JP)

(57) **Abstract**

The present application concerns hair treatment compositions comprising at least one ester of a C8-C22 fatty acid and pentaerythritol in combination with at least one cationic surfactant, the composition having a pH of 1.5 to 7.0.

## Description

### Field of the invention

The present invention relates to hair treatment compositions which contain an ester with excellent conditioning effect and prevent hair from being entangled, imparting softness, smothness, moisture and combability.

### State of the art

Conventional shampoos contain an anionic surfactant as a main component. During shampooing of the hair, not only dirt is removed but also hair-protecting oils, thus decreasing smoothness and combing properties of the hair. To avoid these drawbacks hair treating compositions such as hair rinses and conditioners contain cationic surfactants as main component. However, the use of these surfactants alone cannot sufficiently improve the conditioning effect, various oils, fats, silicones and other components are combined with these surfactants.
Cosmetic compositions with conditioning effect decrease friction of the hair surface and impart softness to the hair at the same time using oils, fats, silicones etc. But sometimes sufficent effects are not achieved with regard to softness and moisture content or hair-styling properties of the hair. One factor influencing the softness of the hair is its water content. Thus, conditioning agents have to influence the water retention properties of the hair. Lanolin which is conventionally used in hair treating agents, satisfies the above mentioned requirements at a considerably high level. Due to its irritant properties it is desirable to find a substitute. JP-B-3-18668 discloses a diester of pentaerythritol in equimolar mixture with a diester of citric acid and a fatty alcohol as substitute for lanolin wax improving the softness of skin. JP-B-59-14520 and JP-A-5-171183 disclose a shampoo or detergent composition comprising a diester of pentaerythritol in combination with a cleaning agent. The diester is said to contribute to the stability of the composition or to improve the dry feeling of the skin.

The object of the present invention is to provide a hair treatment composition which provides an excellent conditioning effect, prevents hair from knotting and imparts softness and moisture retention properties to the hair. Furthermore, the hair-styling properties of hair are improved, even in the absence of anionic surfactants.

### Description of the invention

According to the present invention hair treatment compositions comprising at least one ester of a C8-C22 fatty acid and pentaerythritol and at least one cationic surfactant and having a pH of 1.5 to 7.0 avoid the above mentioned disadvantages and provide the hair with softness and good moisture retention properties. The term "hair treatment composition" used in the present invention is used for any composition suitable for treating hair. Examples thereof include compositions which are washed off such as hair rinses and hair conditioners, or which are left on the hair such as lotions, sprays, emulsions or leave-on conditioners.

The remaining hydroxyl groups in the structure of the ester fo a C8-C22 fatty acid and pentaerythritol used in the present invention are responsible for the hydrating properties of the composition. Particularly when the ester is used in combination with a cationic surfactant, knotting of the hair is prevented, thus improving the combability of the hair, its softness and moisture retention behaviour.

The fatty acids used for esterification with pentaerythritol are saturated or unsaturated, straight chain or branched fatty acids having 8 to 22 carbon atoms, preferably 12 to 22 carbon atoms and more preferably 14 to 22 carbon atoms. It is also possible to use mixtures of fatty acids with different chain lengths, either of natural or synthetic origin.

The ester of the fatty acid with pentaerythritol is preferably a monoester or a diester, more preferably an ester comprising 75 mol% or more based on the ester itself of the diester.

The cosmetic compositions contain the ester of a C8-C22 fatty acid and pentaerythritol in an amount of 0.1 to 20 % by weight, preferably in an amount of 0.5 to 10 % by weight based on the weight of the total composition.

The weight ratio of said pentaerythritol ester and said cationic surfactant is 1:20 to 1:1, preferably 1:10 to 1:2.

As cationic surfactant preferably at least one compound selected from the group consisting of
(a) quaternary ammonium compounds having at least one ester group and at least one or preferably at least two C11-C22-alkyl or alkenyl groups, and
(b) quaternary ammonium compounds having at least one C11-C22 alkyl or alkenyl group is used.
   According to the invention the cosmetic compositions comprise 0.1 to 20 % by weight, preferably 1 to 15 % by weight of the cationic surfactant based on the whole composition.

The hair treating composition of the present invention usually has a pH of 1.5 to 7.0, and preferably of 3.0 to 7.0. If the pH is lower than 1.5, the hair may be damaged during treatment because of swelling. I the pH exceeds 7.0, the desired conditioning effect might not be achieved.
The pH of the hair treating composition can be adjusted using an inorganic or organic acid or an alkali compound which is usually used in cosmetics. Examples of an inorganic alkali compound include sodium hydroxide and potassium hydroxide. Examples for the organic alkali compound include monoethanolamine, diethanolamine, triethanolamine, aminomethylpropanol, arginine and lysine. Examples of the inorganic acid include hydrochloric acid, sulfuric acid and phosphoric acid, as organic acids citric acid, lactic acid and glutamic acid may be used. However, other compounds suitable for cosmetic compositions may also be used.
The hair treating composition furthermore may contain other components suitable for cosmetic compositions such as ail components, surfactants, polymers etc.

### Oil components

Examples for oil components include:
- higher alcohols such as cetyl alcohol, stearyl alcohol, behenyl alcohol or mixtures of different fatty alcohols such as rape-seed or coco fatty alcohol, etc.,
- higher fatty acids such as myristic acid, palmitic acid, stearic acid etc.,
- hydrocarbons such as liquid paraffin, polyisobutene, squalane, etc.,
- waxes such as carnauba wax, candelilla wax, paraffin wax, microcrystalline wax, ceresin, polyethylene wax, etc.;
- ester oils such as animal and vegetable oils, isopropyl myristate, octyldodecyl palmitate, butyl stearate, isononyl isononanoate, etc.;
- triglycerides such as glyceriin triethylhexanoate, glycerin caprylate/caprate, etc.; and
- silicones and silicone derivatives, such as dimethylpolysiloxane, methylphenylpolysiloxane, fatty acid or alkohol or amino or hydroxyalkyl modified silicone, dimethicone, dimethiconol, etc.

### Surfactants

Additionally to the components a) and b) anionic, amphoteric and nonionic surfactants may be used. Examples for anionic surfactants include fatty acid soaps, alkyl sulfates, alkylether sulfates, acylamino acid salts, acyltaurine salts, alkylether phosphates, alkyl succinates, etc. Examples for amphoteric surfactants are alkylbetaines, alkylamidobetaines, alkylimidazolium betaines, alkylamine oxides, etc. As nonionic surfactants the following may be used: alkylethers, alkylesters and polyhydric alcohol esters with a polyoxyalkylene chain, alkylene oxide copolymers, polyhydric alcohol fatty acid esters, glycerin fatty acid esters and ethers, sugar fatty acid esters and ethers, etc.

### Polymers

The following polymers may be used in the hair treating compositions according to the present invention:
- natural polymers such as guar gum, carrageenan, locust bean gum, starch, pectin, xanthan gum, succinoglucan, hyaluronioic acid, chitosan, gelatin, collagen, keratin, etc.
- cellulose polymers such as methylcellulose, carboxymethylcellulose, hydroxyethylcellulose, etc.
- alginic acid based polymers, vinyl polymers, acrylic acid and methacrylic acid based polymers, cationic polymers, amphoteric polymers, urethane polymers, etc.

The hair treating composition further may comprise alcohols, polyhydric alcohols, monosaccharides such as glucose, polysaccharides such as sucrose, vitamins, medicinal actives, animal and vegetable extracts, uv absorbers, powders, preservatives, antioxidants, colorants, perfumes, sequestering agents, etc. the hair treating compositions furthermore are water based.

The hair treating compositions of the present invention can be prepared by mixing the above mentioned components in a conventional manner. For example, the composition can be prepared be heating the oil phase and the aqueous phase seperately to an appropriate temperature, i.e. 80 °C, and then incorporating/emulsifying the aqueous phase into the oil phase. A stirrer or homogenizer may be used.

The present invention will be illustrated by the following examples.
Tables 1 and 2 show hair rinse conditioners and hair styling creams. Their conditioning perfomance was evaluated according to the following organoleptic method.
Twenty female panelists between aged between 18 and 55 used the compositions on their hair grading the different properties (finger combing after rinsing, smoothness of wet hair, softening degree of wet hair, finger combing of dry hair, smoothness of dry hair, softness of dry hair, moisture content of dry hair, hair styling properties of dry hair, gloss of dry hair) according to the following scheme:
- 5: very good
- 3: neither good nor bad
- 1: bad.

### Examples

**Table 1**

| Hair rinse conditioner | Comparative examples | | | | Examples | | | |
|---|---|---|---|---|---|---|---|---|
| Components | 1 | 2 | 3 | 4 | 5 | 6 | 7 | 8 |
| Dicocoylthylhydroxyethylmonium metho- | 2 | | | | 2 | | | |
| sulfate | | | | | | | | |
| Dibehenylethylhydroxyethylmonium | | 2 | | | | 2 | | |
| methosulfate | | | | | | | | |
| Stearyltrimethylammonium chloride | | | 2 | | | | 2 | |
| Distearyldimethylammonium chloride | | | | 2 | | | | 2 |
| Pentaerythrityl distearate | | | | | 2 | | 4 | |
| Pentaerythrityl dibehenate | | | | | | 3 | | 3 |
| Stearyl alcohol | 2 | | 3 | 3 | 3 | | | |
| Behenyl alcohol | 1 | 3 | | | | | | |
| Glycerin monostearate | | 1 | 1 | | | 1 | | |
| Sorbitan monooleate | | | | 1 | | | | 1 |
| POE (20) cetyl ether | 0.2 | 0.2 | | 0.2 | 0.2 | 0.2 | | 0.2 |
| Liquid paraffin | 1 | 1 | | | 1 | 1 | | |
| Triotaoin | | 1 | 1 | | | 1 | 1 | |
| Dimethylpolysiloxane | | | 1 | 1 | | | 1 | 1 |
| Cyclomethicone | | | | 1 | | | | 1 |
| Glycerin | 3 | 3 | 3 | 3 | 3 | 3 | 3 | 3 |
| Sodium hydroxide/Citric acid | q.s. | q.s | q.s. | q.s. | q.s. | q.s. | q.s. | q.s. |
| Water | | | | ad | 100 | | | |
| pH | 3.0 | 3.0 | 4.0 | 4.0 | 3.0 | 3.0 | 4.0 | 4.0 |
| Finger combing after rinsing | 2 | 2 | 3 | 2 | 4 | 5 | 5 | 5 |
| Smoothness of wet hair | 2 | 1 | 2 | 1 | 5 | 4 | 4 | 5 |
| Softening degree of wet hair | 3 | 2 | 3 | 2 | 5 | 5 | 5 | 5 |
| Finger combing of dry hair | 2 | 2 | 2 | 3 | 5 | 5 | 5 | 5 |
| Smoothness of dry hair | 1 | 2 | 2 | 3 | 4 | 5 | 5 | 5 |
| Softness of dry hair | 2 | 1 | 3 | 2 | 4 | 5 | 5 | 4 |
| Moisture content of dry hair | 3 | 2 | 2 | 1 | 5 | 5 | 5 | 5 |
| Styling properties of dry hair | 3 | 2 | 1 | 2 | 5 | 5 | 4 | 4 |
| Gloss of dry hair | 2 | 2 | 3 | 3 | 4 | 5 | 5 | 5 |

**Table 2**

| Hair cream/styling conditioner | Comparative examples | | | | Examples | | | |
|---|---|---|---|---|---|---|---|---|
| Components | 1 | 2 | 3 | 4 | 5 | 6 | 7 | 8 |
| Pentaerythrityl distearate | | | | | 5 | | 10 | |
| Pentaerythrityl dibehenate | | | | | | 5 | | 10 |
| Candelilla wax | 10 | | | 5 | 5 | | | |
| Carnauba wax | | 5 | | | | 5 | | |
| Microcrystalline wax | | 5 | 5 | | | | | |
| Paraffin wax | | | 3 | 5 | | | | |
| Lanolin | 2 | | 2 | 2 | 2 | | | 2 |
| Hydroxystearic acid | 5 | 5 | 5 | 5 | 5 | 5 | 5 | 5 |
| POE (10) cetyl ether | 10 | 10 | 10 | 10 | 10 | 10 | 10 | 10 |
| POE (6) cetyl ether | 2 | 2 | 2 | 2 | 2 | 2 | 2 | 2 |
| Dicapryl carbonate | 10 | | 10 | | 10 | | 10 | |
| Silicone polymer | | 1 | | 1 | | 1 | | 1 |
| Cyclomethicone | | 5 | | 5 | | 5 | | 5 |
| Carboxyvinyl polymer | | | 0.2 | 0.2 | | | 0.2 | 0.2 |
| Polyvinyl pyrrolidone/vinyl acetate | | | 3 | 3 | | | 3 | 3 |
| copolymer | | | | | | | | |
| 1,3-Butanediol | 3 | 3 | 3 | 3 | 3 | 3 | 3 | 3 |
| Sodium hydroxide/citric acid | q.s. | | | | | | | |
| Water | ad 100 | | | | | | | |
| pH | 6.0 | 6.0 | 6.0 | 6.0 | 6.0 | 6.0 | 6.0 | 6.0 |
| Finger combing of dry hair | 2 | 2 | 2 | 3 | 5 | 5 | 5 | 5 |
| Smoothness of dry hair | 1 | 3 | 2 | 3 | 4 | 5 | 5 | 5 |
| Softness of dry hair | 3 | 3 | 2 | 3 | 5 | 5 | 4 | 4 |
| Moisture content of dry hair | 3 | 2 | 3 | 3 | 5 | 5 | 5 | 5 |
| Styling properties of dry hair | 3 | 2 | 3 | 3 | 5 | 5 | 5 | 5 |
| Gloss of dry hair | 2 | 2 | 3 | 3 | 4 | 5 | 5 | 5 |

## Claims

1. A hair treatment composition comprising at least one ester of a C8-C22 fatty acid and pentaerythritol and at least one cationic surfactant and having a pH of 1.5 to 7.0.

2. The hair treatment composition according to claim 1, **characterized in that** said cationic surfactant is at least one compound selected from the group consisting of
(a) quaternary ammonium compounds having at least one ester group and at least one C 11-C22-alkyl or alkenyl group, and
(b) quaternary ammonium compounds having at least one C11-C22 alkyl or alkenyl group.

3. The hair treatment composition according to any of the claims 1 or 2, **characterized in that** said ester of a C8-C22 fatty acid and pentaerythritol is a diester.

4. The hair treatment composition of any of the claims 1 to 3, **characterized in that** the composition contains the ester of a C8-C22 fatty acid and pentaerythritol in an amount of 0.1 to 20 % by weight based on the weight of the total composition.

5. The hair treatment composition of any of the claims 1 to 4, **characterized in that** the composition contains the cationic surfactant in an amount from 0.1 to 20 % by weight based on the weight of the total composition.

6. The hair treatment composition of any of the claims 1 to 5, **characterized in that** the weight ratio of said ester and said cationic surfactant is 1:20 to 1:1.
